# EUROPÄISCHE PATENTSCHRIFT

(11) **EP 1 296 918 B1**
(45) Veröffentlichungstag und Bekanntmachung des Hinweises auf die Patenterteilung: **13.12.2006**
(21) Anmeldenummer: 01969312.6
(22) Anmeldetag: 23.06.2001
(51) Int. Cl.: C07C 33/20, C11B 9/00, C07C 43/164, C07C 43/166, C07C 69/14, C07C 69/007

(54) **Dimethylbenzol-Derivate als Riechstoffe**
Dimethylbenzene derivatives as fragrants
Dérivés de diméthylbenzène en tant qu'agents parfumants

(30) Priorität: 04.07.2000 DE 10032335
(43) Veröffentlichungstag der Anmeldung: 02.04.2003
(73) Patentinhaber: KAO CORPORATION, Chuo-ku Tokyo 103-8210 (JP)
(72) Erfinder: MARKERT, Thomas, 40789 Monheim (DE); FABER, Werner, 47877 Willich (DE); TEN PIERIK, Theo, NL-05916 LE Venlo (NL)
(74) Vertreter: Wolf, Matthias
(86) Internationale Anmeldenummer: PCT/EP2001/007156
(87) Internationale Veröffentlichungsnummer: WO 2002/002494

(56) Entgegenhaltungen:
- EP-A- 0 149 976
- EP-A- 0 283 924
- DE-A- 1 920 672
- DE-A- 19 714 041
- FR-A- 1 538 619
- E. GLYDE, R. TAYLOR: "Electrophilic Aromatic Reactivities via Pyrolysis of 1-Arylethyl Acetates" JOURNAL OF THE CHEMICAL SOCIETY, PERKIN TRANSACTIONS 2., 1977, Seiten 1537-1541, XP002186110 CHEMICAL SOCIETY. LETCHWORTH., GB ISSN: 1472-779X

## Beschreibung

### Gebiet der Erfindung

Die vorliegende Erfindung betrifft neue Dimethylbenzol-Derivate sowie deren Verwendung als Riechstoffe.

### Stand der Technik

Viele natürliche Riechstoffe stehen, gemessen am Bedarf, in völlig unzureichender Menge zur Verfügung. Beispielsweise sind zur Gewinnung von 1 kg Rosenöl 5000 kg Rosenblüten notwendig. Die Folgen sind eine sehr stark limitierte Weltjahresproduktion sowie ein hoher Preis. Es ist daher klar, dass die Riechstoffindustrie einen ständigen Bedarf an neuen Riechstoffen mit interessanten Duftnoten hat. Einerseits kann dadurch die Palette der natürlich verfügbaren Riechstoffe ergänzt werden, andererseits ist es dadurch möglich, die notwendigen Anpassungen an wechselnde modische Geschmacksrichtungen vornehmen zu können. Darüber hinaus wird es auf diese Weise möglich, den ständig steigenden Bedarf an Geruchsverbesserern für Produkte des täglichen Bedarfs wie Kosmetika und Reinigungsmittel decken zu können.

Im Übrigen besteht generell ein ständiger Bedarf an synthethischen Riechstoffen, die sich günstig und mit gleichbleibend hoher Qualität herstellen lassen und die originelle olfaktorische Eigenschaften haben. Insbesondere sollen sie angenehme, möglichst naturnahe und qualitativ neuartige Geruchsprofile von ausreichender Intensität besitzen und in der Lage sein, den Duft von kosmetischen und Verbrauchsgütern vorteilhaft zu beeinflussen. Mit anderen Worten: es besteht ein ständiger Bedarf an Verbindungen, die charakteristische neue Geruchsprofile bei gleichzeitig hoher Haftfestigkeit, Geruchsintensität und Strahlkraft aufweisen.
Phenolketale spezieller Struktur sowie deren Verwendung als Riechstoffe sind in DE 197 14 041 A1 beschrieben.

### Beschreibung der Erfindung

Es wurde gefunden, dass die Verbindungen der allgemeinen Formel (I) die oben genannten Forderungen in jeder Hinsicht ausgezeichnet erfüllen und in vorteilhafter Weise als Riechstoffe mit unterschiedlichen nuancierten Geruchsnoten mit guter Haftfestigkeit eingesetzt werden können.

Gegenstand der vorliegenden Erfindung ist die Verwendung von Dimethylbenzol-Derivaten der allgemeinen Struktur (I) 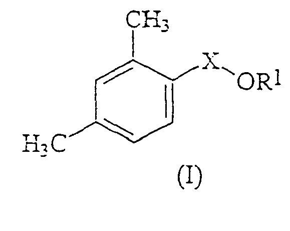 worin X eine Gruppe -(C=CH₂)- oder eine Gruppe -CH(CH₃)- bedeutet mit der Maßgabe, daß
- falls X eine Gruppe -(C=CH₂)- ist, der Rest R¹ einen Alkylrest mit 1 bis 10 C-Atomen bedeutet und
- falls X eine Gruppe -CH(CH₃)- ist, der Rest R¹ Wasserstoff, einen Alkylrest mit 1 bis 10 C-Atomen, der gesättigt oder ungesättigt, geradkettig oder verzweigt sein kann oder einen Acylrest mit 1 bis 10 C-Atomen bedeutet, als Riechstoffe.

Dabei sind folgende Verbindungen bevorzugt:
- 1(1-Ethoxy-vinyl)-2,4-dimethyl-benzol,
- 1-(2,4-Dimethylphenyl)-ethanol,
- 1-(1-Ethoxy-ethyl)-2,4-dimethylbenzol,
- 1-(2,4-Dimethylphenyl)-ethylacetat.

Die Verbindungen (I) zeichnen sich durch eine Geruchscharakteristik aus, in der Anthranilat-Noten sowie blumige und Moschus-Noten dominieren. Sie weisen eine ausgezeichnete Stabilität in Rezepturen der Kosmetik und Gebrauchsparfümerie auf.

Die Herstellung der Verbindungen (I) kann nach bekannten Syntheseverfahren der organischen Chemie erfolgen.

In Parfum-Kompositionen verstärken die Verbindungen (I) die Harmonie und die Ausstrahlung sowie die Natürlichkeit und auch die Haftung, wobei die Dosierung unter Berücksichtigung der übrigen Bestandteile der Komposition auf die jeweils angestrebte Duftnote abgestimmt wird.

Dass die Verbindungen (I) die oben genannten Duftnoten aufweisen, war nicht vorhersehbar und ist damit eine weitere Bestätigung für die allgemeine Erfahrung, dass die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluss der chemischen Struktur auf die Duftwahrnehmung hinreichend erforscht sind und somit also normalerweise nicht vorhergesehen werden kann, ob ein geänderter Aufbau bekannter Riechstoffe überhaupt zur Änderung der olfaktorischen Eigenschaften führt und ob diese Änderungen vom Fachmann positiv oder negativ beurteilt werden.

Die Verbindungen der Formel (I) eignen sich aufgrund ihres Geruchsprofiles insbesondere auch zur Modifizierung und Verstärkung bekannter Kompositionen. Hervorgehoben werden soll insbesondere ihre ausserordentliche Geruchsstärke, die ganz allgemein zur Veredelung der Komposition beiträgt.

Die Verbindungen der Formel (I) lassen sich mit zahlreichen bekannten Riechstoffingredienzien, beispielsweise anderen Riechstoffen natürlichen, synthetischen oder partial-synthetischen Ursprungs, ätherischen Ölen und Pflanzenextrakten kombinieren. Die Palette der natürlichen Riechstoffe kann dabei sowohl leicht-, als auch mittel- und schwerflüchtige Komponenten umfassen. Die Palette der synthetischen Riechstoffe kann Vertreter aus praktisch allen Stoffklassen umfassen.

Beispiele für geeignete Substanzen, mit denen die Verbindungen (I) kombiniert werden können sind insbesondere:
(a) Naturprodukte wie Baummoos-Absolue, Basilikumöl, Agrumenöle wie Bergamotteöl, Mandarinenöl, usw., Mastix-Absolue, Myrtenöl, Palmarosaöl, Patchouliöl, Petitgrainöl, Wermutöl, Myrrheöl, Olibanumöl
(b) Alkohole wie Farnesol, Geraniol, Linalool, Nerol, Phenylethylalkohol, Rhodinol, Zimtalkohol, Sandalore [3-Methyl-5-(2.2.3-trimethylcyclopent-3-en-1-yl)pentan-2-ol], Sandela [3-Isocamphyl-(5)-cyclohexanol],
(c) Aldehyde wie Citral, Helional^{R}, µ-Hexylzimtaldehyd, Hydroxycitronellal, Lilial^{R} [p-tert.-Butyl-µ-methyldihydrozimtaldehyd], Methylnonylacetaldehyd,
(d) Ketone wie Allylionon, µ-Ionon, β-Ionon, Isoraldein, Methylionon,
(e) Ester wie Allylphenoxyacetat, Benzylsalicylat, Cinnamylpropionat, Citronellylacetat, Decylacetat, Dimethylbenzylcarbinylacetat, Ethylacetoacetat, Hexenylisobutyrat, Linalylacetat, Methyldihydrojasmonat, Vetiverylacetat, Cyclohexylsalicylat,
(f) Lactone wie gamma-Undecalacton, 1-Oxaspiro[4.4]nonan-2-on,
sowie verschiedene weitere in der Parfümerie oft benutzte Komponenten wie Moschus- und Sandelholz-Riechstoffe, Indol, p-Menthan-8-thiol-3-on, Methyleugenol und Ambroxan.

Bemerkenswert ist ferner die Art und Weise, wie die Verbindungen der Struktur (I) die Geruchsnoten einer breiten Palette bekannter Kompositionen abrunden und harmonisieren, ohne jedoch in unangenehmer Weise zu dominieren. 1(1-Ethoxy-vinyl)-2,4-dimethyl-benzol, 1-(2,4-Dimethylphenyl)-ethanol, 1-(1-Ethoxy-ethyl)-2,4-dimethylbenzol und 1-(2,4-Dimethylphenyl)-ethylacetat sind in dieser Hinsicht ganz besonders hervorzuheben.

Die einsetzbaren Anteile der erfindungsgemäßen Verbindungen (I) oder deren Gemische in Riechstoffkompositionen bewegen sich von etwa 1-70 Gew. %, bezogen auf die gesamte Mischung. Gemische der erfindungsgemäßen Verbindungen (I) sowie Kompositionen dieser Art können sowohl zur Parfümierung kosmetischer Präparate wie Lotionen, Cremes, Shampoos, Seifen, Salben, Pudern, Aerosolen, Zahnpasten, Mundwässern, Deodorantien als auch in der alkoholischen Parfümerie (z.B. Eau de Cologne, Eau de Toilette, Extraits) verwendet werden. Ebenso besteht eine Einsatzmöglichkeit zur Parfümierung technischer Produkte wie Wasch- und Reinigungsmittel, Weichspüler und Textilbehandlungsmittel. Zur Parfümierung dieser verschiedenen Produkte werden diesen die Kompositionen in einer olfaktorisch wirksamen Menge, insbesondere in einer Konzentration von 0,05 - 2 Gew. % - bezogen auf das gesamte Produkt - zugesetzt. Diese Werte sind jedoch keine beschränkenden Grenzwerte, da der erfahrenen Parfümeur auch mit noch geringeren Konzentrationen noch Effekte erzielen oder mit noch höheren Dosierungen neuartige Komplexe aufbauen kann.

### Beispiele

### Beispiel 1: Herstellung von 1-(2,4-Dimethylphenyl)-ethanol

**Ansatz:** a) 296 g (2 mol) 2,4-Dimethylacetophenon (Fa. Cognis)
   b) 318 g (2,2 mol) Vitride (70%ig in Toluol)
   c) 300 ml Toluol, wasserfrei
**Apparatur:** 2 l Vierhalskolben mit Rührer, Thermometer und Rückflußkühler, Inertgas.
**Ausführung:** Unter Stickstoff wurden 296 g 2,4-Dimethylacetophenon und 300 ml über Molsieb getrocknetes Toluol im Reaktionskolben gemischt. Anschließend wurde unter starkem Rühren und Kühlung mit einem Eisbad innerhalb 3,5 Stunden 318 g Vitride-Lösung zugetropft. Die Temperatur der Mischung stieg dabei auf 48°C an. Die gaschromatographische Umsatzkontrolle zeigte 3% Edukt und 92,1% Produkt an. Es wurde noch 2 Stunden bei Raumtemperatur nachgerührt.
**Aufarbeitung:** Nach Abkühlen auf Raumtemperatur wurden zu der unter Stickstoff gerührten Mischung vorsichtig 600 ml 10%ige Natronlauge in ca. 3 Stunden zudosiert. Dabei wurde anfangs eine starke Wasserstoffentwicklung beobachtet. Das Gemisch wurde in einen 5 Liter Scheidetrichter überführt und die Wasserphase abgetrennt. Die Toluolphase wurde 1 mal mit 10%iger Natronlauge und 2 mal mit gesättigter Kochsalzlösung gewaschen, über Natriumsulfat getrocknet und am Rotationsverdampfer eingeengt.
125 g Rohprodukt wurden an einer 20cm-Vigreuxkolonne destilliert. Es wurden 108 g Destillat mit einer GC-Reinheit von 93%, bei einem Siedepunkt von 83 - 84°C/0.15 mbar erhalten.
**Analytik:** Das ¹H-NMR-Spektrum (280 MHz, in CDCl₃) zeigte 1 Dublett (1 Methyl-Gruppe) bei chemischen Verschiebungen von 1,4 ppm und 2 Singuletts (je 1 Methyl-Gruppe) bei 2,3 ppm. 1 Multiplett (1 H) erschien bei 5,1 ppm und die 3 aromatischen Protonen bei 6.9, 7,0 und 7,4 ppm.
Das IR-Spektrum (Film zwischen NaCl) zeigte C-O-Banden bei 1007, 1078 und 1128 cm⁻¹ und ein breites Signal für die -OH Schwingung bei 3345 cm⁻¹ mit einer Schulter bei 3550 cm⁻¹.
**Geruchscharakteristik:** Angeruch: animalisch, Indol-, Salicylat-Noten; Nachgeruch (nach 24 Stunden am Riechstreifen): Indol, animalisch, Anthranilat-, Salicylat-Noten.

### Beispiel 2: Herstellung von 1-(1-Ethoxy-ethyl)-2,4-dimethylbenzol

**Ansatz:** a) 111,0 g (0,5 mol) 2,4-Dimethylacetophenondiethylketal
   (hergestellt nach WO 98/45236)
   b) 100 ml Ethanol, wasserfrei
   c) 2,5 g 5%Palladium/Kohle
**Apparatur:** 11 Stahlautoklaveneinsatz, Hubrührhochdruckautoklav
**Ausführung:** In einen 11 Stahlautoklaveneinsatz wurden die Komponenten a), b) und c) nacheinander unter Stickstoff eingewogen. Im Autoklaven wurde die Mischung zunächst mit Stickstoff gespült und anschließend mit 60 bar Wasserstoff beaufschlagt. Der Autoklav wurde auf 100°C beheizt und anfangs halbstündlich später stündlich mit Wasserstoff auf 100 bar gebracht. Nach 4,5 Stunden wurde keine Wasserstoffaufnahme mehr beobachtet, es wurde noch 0,5 Stunden nachgeheizt, abgekühlt und entspannt. Der Katalysator wurde abfiltriert und das Reaktionsgemisch am Rotationsverdampfer eingeengt. 60,4 g Rohprodukt wurden an einer 20cm-Vigreuxkolonne destilliert. Es wurden 19,6 g 1-Ethyl-2,4-dimethylbenzol (Sdp. 33°C /0.07 mbar, Reinheit: 96,7%) als Nebenprodukt und 22,5 g Hauptprodukt [1-(1-Ethoxyethyl)-2,4-dimethylbenzol] mit einer GC-Reinheit von 92,2%, bei einem Siedepunkt von 45-50°C/0.07 mbar erhalten.
**Analytik:** Das ¹H-NMR-Spektrum (280 MHz, in CDCl₃) zeigte 2 Singuletts (2 Methyl-Gruppen) bei chemischen Verschiebungen von 2,2 ppm, 1 Dublett (1 Methyl-Gruppe) bei 1,4 und ein Triplett (1 Methyl-Gruppe) bei 1,1 ppm. Bei 3,3 ppm erschien ein Dublett vom Quadruplett (1 Methylen-Gruppe) und bei 4,6 ppm ein Quadruplett (1H). Die 3 aromatischen Protonen ergaben Resonanzen mit dem typischen Kopplungsmuster der 1,2,4-Substitution bei 6,9; 7,0 und 7,3 ppm.
Das IR-Spektrum (Film zw.NaCl) zeigte breite Etherbanden bei 1065, 1093, 1118 und 1154 cm⁻¹.
**Geruchscharakteristik:** Angeruch: blumig, fruchtig, Eau de Cologne, Moschusketon-Noten; im Nachgeruch Anthranilat-, Salicylat-, Indol-Noten.

### Beispiel 3: Herstellung von 1-(2,4-Dimethylphenyl)-ethylacetat

**Ansatz:** a) 90,0 g (0,6 mol) 1-(2,4-Dimethylpenyl)ethanol
   (hergestellt nach Beispiel 1)
   b) 200 g Acetanhydrid
   c) 0,5 g 4-N,N-Dimethylaminopyridin (Fa. Aldrich)
**Apparatur**: 1 L Vierhalskolben mit Rührer, Innenthermometer, Rückflußkühler und Tropftrichter.
**Ausführung:** a) und c) wurden in den Reaktor eingewogen und unter Rühren bei Raumtemperatur in 40 Minuten b) kontinuierlich zugetropft. Dabei wurde ein Temperaturanstieg der Mischung auf 40°C beobachtet. Durch Kühlung mit einem Wasserbad wurde die Temperatur der Reaktionsmischung bis zur vollständigen Zugabe von b) auf 40°C gehalten. Nach vollständiger Zugabe von b) wurde 3 Stunden gerührt und die Mischung über Nacht bei Raumtemperatur stehen gelassen.
**Aufarbeitung:** Überschüssiges Acetanhydrid und Essigsäure wurden im Wasserstrahlvakuum abdestilliert. Der Rückstand wurde mit 100 ml Cyclohexan verdünnt und mit gesättigter Natriumbicarbonatlösung und mit Wasser neutral gewaschen, über Natriumsulfat getrocknet und am Rotationsverdampfer eingeengt.
95 g Rohprodukt wurden an einer 20cm Vigreuxkolonne destilliert. Es wurden 56 g Destillat erhalten, die Hauptmenge hatte eine GC-Reinheit von 97,9% bei einem Siedepunkt von 77 -80°C/0.15 mbar.
**Analytik:** Das ¹H-NMR-Spektrum (280 MHz in CDCl₃) zeigte 1 Dublett (1 Methyl-Gruppe) bei einer chemischen Verschiebung von 1,5 ppm und bei 2,0; 2,3 und 2,35 ppm 3 Singuletts (3 Methyl-Gruppen). Ein Quadruplett (1H) erschien bei 6,0 ppm und die 3 aromatische Protonen bei 6,9;7,0 und 7,25 ppm.
Das IR-Spektrum (Film zw. NaCl) zeigte scharfe Schwingungsbanden bei 1236 (C-O) und 1738 (C=O) cm⁻¹.
**Geruchscharakteristik:** Im Angeruch: Jasmon-, Anthranilat-, Moschus-, Anis-Noten, im Nachgeruch (nach 24 Stunden am Riechstreifen) schwach holzig, Jasmon-Note.

### Beispiel 4: Herstellung von 1(1-Ethoxy-vinyl)-2,4-dimethyl-benzol

**Ansatz:** a) 100,1 g 2,4-Dimethylacetophenon (Fa. Cognis)
   b) 110,9 g Triethylorthoformiat
   d) 0,21 g Schwefelsäure, konz.
   e) 191,4 g Ethanol, 99%
**Apparatur:** 1 l Vierhalskolben mit Rührer, Thermometer und Intensivkühler, Inertgas.
**Ausführung:** Unter Stickstoff und Feuchtigkeitsausschluß wurden die Komponenten a), b) und d) vorgelegt. Die Komponente c) wurde vorsichtig unter starkem Rühren zugegeben. Das Gemisch wurde 3 Stunden bei Raumtemperatur gerührt und anschlie-ßend mit 0,54 g Triethanolamin (pH = 7) neutralisiert. Darüber hinaus wurde mit weiteren 0,44 g Triethanolamin auf pH 8 eingestellt.
**Aufarbeitung**: Das Reaktionsgemisch wurde am Rotationsverdampfer von Ethanol, Ameisensäureester und Triethylorthoformiat befreit.
149,1 g Rohprodukt wurden an einer 20 cm Vigreuxkolonne destilliert, die Hauptmenge (48,2 g, 97,8%) destillierte bei 72-73 °C/0.03 mbar über.
Ausbeute: 82,4% der Theorie
**Analytik**: Das ¹H-NMR (400 MHz, in d6-DMSO) zeigte 1 Triplett bei 1,3 ppm (1 Methyl-Gruppe) und 2 Singuletts bei 2,2 ppm (2 Methyl-Gruppen). Außerdem erschien ein Quadruplett (1 Methylen-Gruppe) bei 3,8 ppm. Die beiden olefinischen Protonen erschienen als Singuletts bei 4,1 und 4,3 ppm und die 3 aromatischen Protonen mit dem charakteristischen Aufspaltungsmuster bei 6,9, 7,0 und 7,1 ppm.
**Geruchscharakteristik**: im Angeruch blumig, Orangenblüte, Honig-, Anthranilat-, Indol-, Nitromoschus-Noten; im Nachgeruch (nach 24 Stunden am Riechstreifen) Salicylat-, Anthranilat-Note.

## Patentansprüche

1. Verwendung von Dimethylbenzol-Derivaten der allgemeinen Struktur (I) 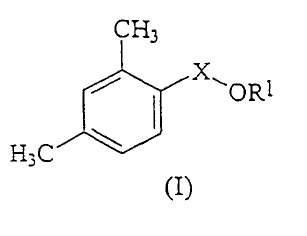 worin X eine Gruppe -(C=CH₂)- oder eine Gruppe -CH(CH₃)- bedeutet mit der Maßgabe, daß
• falls X eine Gruppe -(C=CH₂)- ist, der Rest R¹ einen Alkylrest mit 1 bis 10 C-Atomen bedeutet und
• falls X eine Gruppe -CH(CH₃)- ist, der Rest R¹ Wasserstoff, einen Alkylrest mit 1 bis 10 C-Atomen, der gesättigt oder ungesättigt, geradkettig oder verzweigt sein kann oder einen Acylrest mit 1 bis 10 C-Atomen bedeutet,
als Riechstoffe.

2. Riechstoff-Kompositionen umfassend eine oder mehrere Verbindungen der Formel (I) wie definiert in Anspruch 1 und eine oder mehrere Verbindungen, die ausgewählt sind aus anderen Riechstoffen natürlichen, synthetischen oder partial-synthetischen Ursprungs, ätherischen Ölen und Pflanzenextrakten, wobei die Verbindungen (I) in einer Menge von 1 bis 70 Gew.-% bezogen auf die gesamte Komposition enthalten sind.

## Claims

1. Use of dimethylbenzene derivatives of the general structure (I) 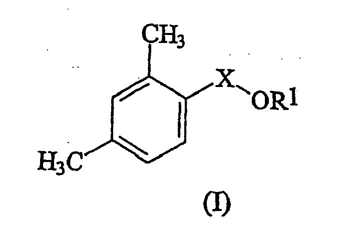 wherein X denotes a group -(C=CH₂)- or a group -CH(CH₃)- with the proviso that
• if X is a group -(C=CH₂)-, the radical R¹ denotes an alkyl radical having 1 to 10 C atoms and
• if X is a group -CH(CH₃)-, the radical R¹ denotes hydrogen, an alkyl radical having 1 to 10 C atoms, which may be saturated or unsaturated, straight-chain or branched, or an acyl radical having 1 to 10 C atoms,
as perfumes.

2. Perfume compositions comprising one or more compounds of the formula (I), as defined in claim 1, and one or more compounds which are selected from other perfumes of natural, synthetic or partially synthetic origin, essential oils and plant extracts, wherein the compounds (I) are present in a quantity of 1 to 70 wt.% relative to the total composition.

## Revendications

1. Utilisation de dérivés de diméthylbenzène de la structure générale (I) 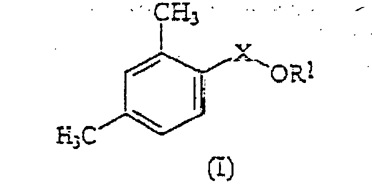 dans laquelle X signifie un groupe -(C=CH₂)- ou un groupe -CH(CH₃)- à condition que
- au cas où X est un groupe -(C=CH₂)-, le radical R¹ signifie un radical alkyle avec 1 à 10 atomes de C et
- au cas où X est un groupe -CH(CH₃)-, le radical R¹ signifie hydrogène, un radical alkyle avec 1 à 10 atomes de C qui peut être saturé ou insaturé, linéaire ou ramifié ou un radical acyle avec 1 à 10 atomes de C,
comme parfums.

2. Compositions de parfums comprenant un ou plusieurs composés de la formule (I) comme défini à la revendication 1, et un ou plusieurs composés sélectionnés parmi d'autres parfums d'origine naturelle, synthétique ou partiellement synthétique, des huiles essentielles et des extraits végétaux, les composés (I) étant contenus en une quantité de 1 à 70 % en poids par rapport à la composition totale.
